(19) Europäisches Patentamt
European Patent Office
Office européen des brevets

(11) Publication number: 0 042 639
B1

(12) # EUROPEAN PATENT SPECIFICATION

(45) Date of publication of patent specification: 22.08.84

(21) Application number: 81200619.5

(22) Date of filing: 04.06.81

(51) Int. Cl.$^3$: C 07 D 207/16, C 07 D 207/404, C 07 D 207/452, C 07 D 211/60, C 07 D 211/88, C 07 D 401/04, C 07 D 403/04, C 07 D 417/04 // A61K31/40, A61K31/41, A61K31/425, A61K31/445, A61K31/47

(54) **Azido, imido, amido and amino derivatives of mercaptoacyl prolines and pipecolic acids having hypotensive activity.**

(30) Priority: 23.06.80 US 162341
23.07.80 US 171565

(43) Date of publication of application:
30.12.81 Bulletin 81/52

(45) Publication of the grant of the patent:
22.08.84 Bulletin 84/34

(84) Designated Contracting States:
AT BE CH DE FR GB IT LI LU NL SE

(56) References cited:
GB-A-2 027 205
US-A-4 046 889
US-A-4 105 776
US-A-4 198 515

(73) Proprietor: E.R. Squibb & Sons, Inc.
Lawrenceville-Princeton Road
Princeton, N.J. 08540 (US)

(72) Inventor: Krapcho, John
150 DeMott Lane
Somerset New Jersey (US)
Inventor: Wade, Peter C.
7873 Kellogg Creek Drive
Mentor Ohio (US)
Inventor: Petrillo, Edward W., Jr.
433 Sked Street
Pennington New Jersey (US)
Inventor: Weisenborn, Frank L.
2 Maddock Road
Titusville New Jersey (US)

(74) Representative: Maiffret, Bernard et al
Law Offices of William J. Rezac 49, avenue
Franklin D. Roosevelt
F-75008 Paris (FR)

EP 0 042 639 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Compounds having the formula

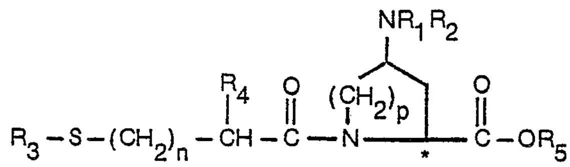

I

and salts thereof, and the symmetrical dimer thereof, inhibit the action of angiotensin converting enzyme, and are therefore, useful for the treatment of hypertension. In formula I, and throughout the specification, the symbols are as defined below.

The group —$NR_1R_2$ is azido or $R_1$ and $R_2$ are the same or different and are hydrogen, alkyl, cycloalkyl, 1-adamantyl, aryl, arylalkyl, alkylcarbonyl, arylcarbonyl, aralkylcarbonyl, alkylsulfonyl, arylsulfonyl or arylvinylcarbonyl (aryl-CH=CH—CO—), or together with the nitrogen atom to which they are attached $R_1$ and $R_2$ are 1-pyrrolidinyl, 1-piperidinyl, 1-homopiperidinyl, 4-morpholinyl, 4-alkyl-1-piperazinyl, 4-aryl-1-piperazinyl, 1-imidazolyl, 1-pyrrolidinyl-2,5-dione(succinimido), 3-alkyl-1-pyrrolidinyl-2,5-dione, 3-aryl-1-pyrrolidinyl-2,5-dione, 1-piperidinyl-2,6-dione, 3-alkyl-1-piperidinyl-2,6-dione, 3-aryl-1-piperidinyl-2,6-dione, 2H-isoindol-2-yl-1,3-dione (phthalimido), hexahydro-2H-isoindol-2-yl-1,3-dione (hexahydrophthalimido), 2,5-dihydro-2,5-dioxo-1H-pyrrol-1-yl(maleimido), 1,1,3-trioxo-1,2-benzisothiazol-2(3H)-yl/(2-saccharinyl), or 1,3-dihydro-1,3-dioxo-2H-benz[de]isoquinolin-2-yl/(1,8-naphthalenedicarboximido);

$R_3$ is hydrogen, alkyl, aryl, arylalkyl, or a hydrolyzable acyl protecting group such as alkanoyl or arylcarbonyl;

$R_4$ is hydrogen, alkyl, alkylthio or trifluoromethyl;

$R_5$ is hydrogen, alkyl or arylalkyl;

n is 0, 1 or 2; and

p is 1 or 2.

The term "aryl", as used throughout the specification either by itself or as part of a larger group, refers to phenyl, 1-naphthyl, 2-naphthyl or phenyl substituted with halogen, alkyl, alkoxy, alkylthio, hydroxy, alkanoyl, nitro, amino, dialkylamino, phenyl or trifluoromethyl groups. Phenyl and monosubstituted phenyl are the preferred aryl groups; phenyl is the most preferred group.

The term "cycloalkyl", as used throughout the specification either by itself or as part of a larger group, refers to cycloalkyl groups having 3 to 7 carbon atoms.

The term "alkyl", as used throughout the specification either by itself or as part of a larger group, refers to groups having 1 to 8 carbon atoms. Alkyl groups having 1 to 3 carbon atoms are preferred.

The term "alkoxy", as used throughout the specification either by itself or as part of a larger group, refers to groups having 1 to 8 carbon atoms. Alkoxy groups having 1 to 3 carbon atoms are preferred.

The term "halogen", as used throughout the specification either by itself or as part of a larger group, refers to flourine, chlorine, bromine and iodine. The preferred halogen groups are fluorine and chlorine.

The term "alkanoyl", as used throughout the specification either by itself or as part of a larger group, refers to groups having 1 to 9 carbon atoms.

The asterisk in formula I indicates a center of asymmetry in the ring. In the instance wherein p is 1 this asymmetric center is preferably in the L-configuration (as in proline). In the instance where the ring is that of pipecolic acid (p is 2) this asymmetric center is preferably in the DL- or L-configuration.

The carbon atom to which the group $NR_1R_2$ is attached is another asymmetric center. Depending on the definition of $R_4$, the sulfur containing side-chain may also contain an asymmetric center. The product of formula I, therefore, exists in stereoisomeric forms and as racemic mixtures thereof. All are within the scope of this invention. The synthesis described below can utilize the racemate or one of the enantiomers as starting materials. When one of the starting materials is chiral and the other is racemic, the stereoisomers obtained in the final product can be separated by conventional chromatographic or fractional crystallization methods. Preferably, if there is an asymmetric center in the sulfur containing side-chain, it is in the D-configuration.

Antihypertensive proline derivatives falling within the formula I as defined above, but having 4-hydroxy or 4-methyl instead of 4—$NR_1R_2$, are known from US—A—4 105 776 (e.g. examples 5 and 22). The 2,3-didehydro-derivatives known from US—A—4 198 515 show further 4-substituents such as chloro, fluoro and methoxy (e.g. examples 12, 13, 14). The prior art does not teach, however, that the antihypertensive activity is retained if the substituent in the 4-position is an azido, imido, amido or amino group.

The compound of formula I, and salts thereof, and the dimer thereof, are useful as hypotensive agents. They inhibit the conversion of the decapeptide angiotensin I to angiotensin II and, therefore, are useful in reducing or relieving angiotensin related hypertension. The action of the enzyme renin on

angiotensinogen, a pseudoglobulin in blood plasma, produces angiotensin I. Angiotensin I is converted by angiotensin converting enzyme (ACE) to angiotensin II. The latter is an active pressor substance which has been implicated as the causative agent in various forms of hypertension in various mammalian species, *e.g.,* rats and dogs. The compounds of this invention intervene in the angiotensinogen → (renin) → angiotensin I → (ACE) → angiotensin II sequence by inhibiting angiotension converting enzyme and reducing or eliminating the formation of the pressor substance angiotensin II. Thus by the administration of a composition containing one or a combination of the compounds of this invention, angiotensin dependent hypertension in the species of mammal (e.g., humans) suffering therefrom is alleviated. A single dose, or preferably two to four dividied daily doses, provided on a basis of about 0.1 to 100 mg. per kilogram of body weight per day, preferably about 1 to 15 mg. per kilogram of body weight per day is appropriate to reduce blood pressure. The substance is preferably administered orally, but parenteral routes such as the subcutaneous, intramuscular, intravenous or intraperitoneal routes can also be employed.

The compounds of this invention can also be formulated in combination with a diuretic for the treatment of hypertension. A combination product comprising a compound of this invention and a diuretic can be administered in an effective amount which comprises a total daily dosage of about 30 to 600 mg., preferably about 30 to 300 mg. of a compound of this invention, and about 15 to 300 mg., preferably about 15 to 200 mg. of the diuretic, to a mammalian species in need thereof. Exemplary of the diuretics contemplated for use in combination with a compound of this invention are the thiazide diuretics, *e.g.,* chlorthiazide, hydrochlorthiazide, flumethiazide, hydroflumethiazide, bendroflumethiazide, methychlotiazide, trichlormethiazide, polythiazide or benzthiazide as well as ethacrynic acid, ticrynafen, chlorthalidone, furosemide, musolimine, bumetanide, triamterene, amiloride and spironolactone and salts of such compounds.

The compounds of formula I can be formulated for use in the reduction of blood pressure in compositions such as tablets, capsules or elixirs for oral administration or in sterile solutions of suspensions for parenteral administration. About 10 to 500 mg. of a compound or mixture of compounds of formula I is compounded with a physiologically acceptable vehicle, carrier, excipient, binder, preservative, stabilizer, flavor, etc., in a unit dosage form as called for by accepted pharmaceutical practice. The amount of active substance in these compositions or preparations is such that a suitable dosage in the range indicated is obtained.

The compounds of this invention can be prepared using 4-hydroxy-L-proline or 4-hydroxypipecolic acid as the starting material. In converting these 4-hydroxy compounds to the corresponding 4-amino, amido or imido compounds it is necessary to first protect the nitrogen atom and the carboxyl group. A protecting group such as carbobenzyloxy, tosyl, acetyl, benzoyl, etc., can first be attached to the nitrogen atom. For example, as described in the literature (see *Journal of the American Chemical Society, 86,* 4709 (1964)), 4-hdroxy-L-proline can be reacted with *p*-toluenesulfonyl chloride to yield 4 - hydroxy - 1 - [(4 - methylphenyl)sulfonyl] - L - proline. The carboxyl group of the 4-hydroxy-N-tosyl-L-proline or pipecolic acid can be protected by esterification, *e.g.,* by reaction with a diazoalkane (see *Journal of the American Chemical Society, 79,* 191 (1957)).

The 4-hydroxy substituent of the resulting 4-hydroxy-N-tosyl-L-proline or pipecolic acid ester can be converted to a leaving group using art-recognized procedures. For example, reaction of the 4-hydroxy compound with *p*-toluenesulfonyl chloride following the procedure described in the *Journal of the Americal Chemical Soceity, 79,* 191 (1957) yields 4,N-ditosyl-L-proline, alkyl ester.

Displacement of the 4-protecting group with a compound having the formula

$$HNR_1'R_2', \qquad II$$

or an alkali metal salt thereof, is accomplished with inversion of the stereoisomerism of the compound. Therefore, if 4-*trans*-hydroxyproline is used as the starting material the product will have the *cis* configuration, and if 4-*cis*-hydroxyproline is used as the starting material the product will have the *trans* configuration. The resulting compound will have the formula

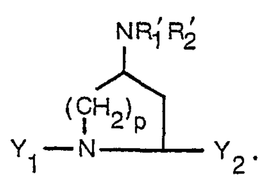

III

In formulas II and III, and throughout the specification, the symbols $R_1'$ and $R_2'$ have the same definitions as $R_1$ and $R_2$ respectively, except $R_1'$ and $R_2'$ cannot both be hydrogen; the symbols $Y_1$ and $Y_2$ are a protecting group and a protected carboxyl group respectively.

Prior to acylating an intermediate of formula III to obtain a product of formula I, the protecting group is removed from the nitrogen atom and can optionally be removed from the carboxyl group. Techniques for deprotection are well known in the art. If the carboxyl group has been esterified to

protect it, the protecting group can be removed using conventional saponification techniques. If the nitrogen atom has been protected with a tosyl group, that group can be removed by treating the compound with a solution of hydrogen bromide. The deprotected amino acid has the formula

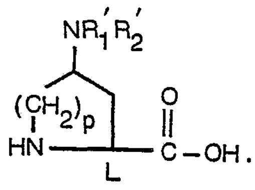

IV

The compounds of formula I ($R_1$ and $R_2$ other than hydrogen) can be obtained by acylating an intermediate of formula IV with an acid having the formula

$$R_3'\text{—}S\text{—}(CH_2)_n\text{—}\overset{R_4}{\overset{|}{C}}H\text{—}COOH, \qquad V$$

wherein $R_3'$ is alkyl, aryl, arylalkyl or a hydrolyzable acyl protecting group. The acylation reaction is run in the presence of a coupling agent such as dicyclohexylcarbodiimide, or the acid can be activated by formation of its mixed anhydride, symmetrical anhydride, acid halide (preferably acid chloride) or acid ester, or by the use of Woodward reagent, K, N-ethoxycarbonyl-2-ethoxy-1,2-dihydroquinoline or the like. For a review of these methods of acylation reference can be made to *Methoden der Organischen Chemie* (Houben-Weyl), Vol. XV, part II, page 1 et seq. (1974).

To prepare a compound of formula I wherein $R_3$ is hydrogen, a corresponding product of formula I wherein $R_3$ is a hydrolyzable acyl protecting group can be treated with base, such as potassium hydroxide or ammonia, in water or other suitable solvent.

The compounds of this invention wherein one, or both, of $R_1$ and $R_2$ are hydrogen can be obtained from the corresponding product of formula I wherein one, or both, of $R_1$ and $R_2$ are phenylmethyl. The phenylmethyl group can be removed by treatment with sodium and liquid ammonia.

An alternative synthesis for the preparation of the compounds of this invention, wherein at least one of $R_1$ and $R_2$ is alkylcarbonyl, arylcarbonyl, arylalkylcarbonyl, alkylsulfonyl, arylsulfonyl or arylvinylcarbonyl also utilizes 4-hydroxy-L-proline or pipecolic acid as the starting material. The nitrogen atom is first protected, *e.g.,* by reaction with benzyl chloroformate in the presence of an organic amine (*e.g.,* triethylamine). The reaction can be run in an inert solvent and yields N-benzyloxycarbonyl-4-hydroxy-L-proline or pipecolic acid. Protection of the carboxyl group of the N-protected compound can be accomplished by reaction with a diazoalkane or by heating with benzyl chloride in the presence of an organic amine following the procedure described in the *Canadian Journal of Biochemistry and Physiology,* 37, 583 (1959). The resulting N-benzyloxycarbonyl-4-hydroxy-L-proline or pipecolic acid ester can be reacted with a compound having the formula

$$HNR_1''R_2''$$

in the presence of diethylazodicarboxylate and triphenylphosphine to yield N-benzyloxycarbonyl-4-($NR_1''R_2''$)-L-proline or pipecolic acid ester, which can in turn be deprotected by catalytic hydrogenation using, for example a palladium catalyst. Acylation of 4-($NR_1''R_2''$)-L-proline, or pipecolic acid (using the procedures described above for the acylation of an intermediate of formula III) yields the corresponding product of formula I. The symbols $R_1''$ and $R_2''$ are the same or different and are hydrogen, alkyl, cycloalkyl, 1-adamantyl, aryl, arylalkyl, alkylcarbonyl, arylcarbonyl, arylalkylcarbonyl, alkylsulfonyl, arylsulfonyl or arylvinylcarbonyl, with the proviso that at least one of $R_1''$ and $R_2''$ are one of the acyl or substituted sulfonyl groups.

A preferred synthesis for the products of formula I wherein $R_1$ and $R_2$ each is hydrogen comprises first preparing the corresponding product of formula I wherein —$NR_1R_2$ is phthalimido. Reaction of this product with hydrazine in an inert solvent yields the corresponding 4-amino product.

Still another procedure for preparing the compounds of this invention wherein $R_1$ is hydrogen and $R_2$ is alkylcarbonyl, arylcarbonyl, arylalkylcarbonyl or arylvinylcarbonyl comprises first preparing 4-azido-1-tosyl-L-proline or pipecolic acid (see the *Australian Journal of Chemistry, 20,* 1493 (1967)). This compound can be reduced using catalytic hydrogenation to obtain 4-amino-1-tosyl-L-proline or pipecolic acid, alkyl ester. Acylation of this compound yields the corresponding 4-acylamino-1-tosyl-L-proline or pipecolic acid, alkyl ester which can be deprotected and then acylated with an acid of formula V following the procedure described above.

Still another procedure for preparing the compounds of this invention utilizes the 4-hydroxyprolines or 4-hydroxypipecolic acids having the formula

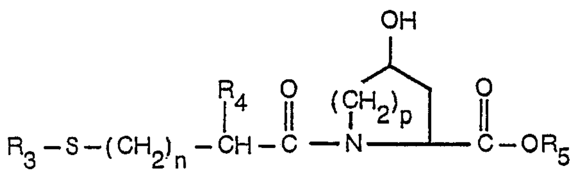

VII

wherein $R_3$ and $R_5$ are other than hdyrogen, as starting materials. The compounds of formula VII are disclosed in United States patent 4,105,776. Reaction of a compound of formula VII wherein the 4-hydroxyl group is first converted to a leaving group such as tosylate, methanesulfonate or triflate ($CF_3$—$SO_2$) with a compound of the formula

$$HNR_1R_2, \qquad VIII$$

or a sodium or potassium salt thereof, gives the corresponding product of formula I.

Still another procedure for preparing the compounds of this invention wherein at least one of $R_1$ and $R_2$ is alkylcarbonyl, arylcarbonyl, arylalkylcarbonyl, alkylsulfonyl, arylsulfonyl or arylvinylcarbonyl comprises reacting a compound of formula VII with a compound of formula VI in the presence of triphenylphosphine and diethylazodicarboxylate. This procedure can also be used to prepare compounds of formula I wherein —$NR_1R_2$ is the azido (—$N_3$) group. Thus, by replacing the formula VI compound with a hydrazoic acid, a compound of formula VII is obtained wherein the hdyroxyl group is replaced with the azido group, i.e.,

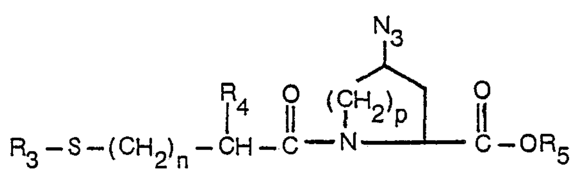

VIIIa

$R_3$ and $R_5$ being other than hydrogen, which can then be hydrolyzed, where $R_3$ is a hydrolyzable acyl group, to form products wherein $R_3$ adn $R_5$ are hydrogen. Alternatively, the azido compound can be prepared by reacting the formula IV compound wherein $NR_1'R_2'$ is the azido group with the acid of formula V followed by conventional hydrolysis where $R_3'$ is a hydrolyzable protecting group to form the product wherein $R_3'$ is hydrogen.

The compounds of this invention form basic salts with various inorganic and organic bases which are also within the scope of the invention. Such salts include ammonium salts, alkali metal salts like sodium and potassium salts (which are preferred), alkaline earth metal salts like the calcium and magnesium salts, salts with organic bases, *e.g.,* dicyclohexylamine salt, benzathine, N-methyl-D-glucamine, hydrabamine salts, salts with amino acids like arginine, lysine and the like. The non-toxic, physiologically acceptable salts are preferred, although other salts are also useful, *e.g.,* in isolating or purifying the product.

The salts are formed in conventional manner by reacting the free acid form of the product with one or more equivalents of the appropriate base providing the desired cation in a solvent or medium in which the salt is insoluble, or in water and removing the water by freeze drying. By neutralizing the salt with an an insoluble acid like a cation exchange resin in the hydrogen form (e.g., polystyrene sulfonic acid resin like Dowex 50) or with an aqueous acid and extraction with an organic solvent, e.g., ethyl acetate, dichloromethane or the like, the free acid form can be obtained, and, if desired, another salt formed.

The symmetrical dimer of a product of formula I, *i.e.,* compound having the formula

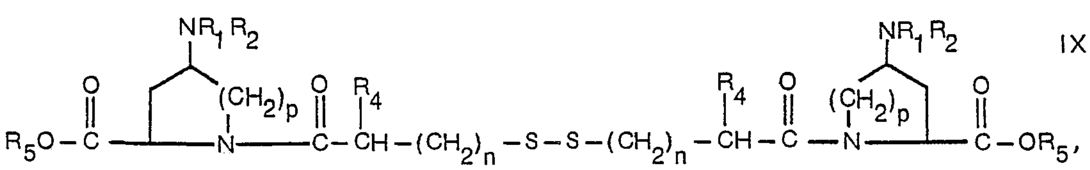

IX

is also contemplated as an integral part of this invention. It is readily prepared from the corresponding product of formula I wherein $R_3$ is hydrogen by direct oxidation with iodine.

Compounds of formula I wherein p is 1 are preferred. Also preferred as compounds of formula I wherein n is 1 and $R_4$ is methyl.

The following examples are specific embodiments of this invention.

Example 1

[1(S),4S]-4-(Benzoylamino)-1-(3-mercapto-2-methyl-1-oxopropyl)-L-proline

A) *(R)-4-Hydroxy-1-[(4-methylphenyl)sulfonyl]-L-proline*

A stirred solution of 52.45 g (0.40 mole) of *trans*-4-hydroxy-L-proline in 500 ml of sodium hydroxide is treated with 85.79 g (0.45 mole) of *p*-toluenesulfonyl chloride in 500 ml of ether at room temperature. Stirring is continued for 6 hours, the layers are separated and the aqueous phase is cooled to 5°C in an ice-bath. The mixture is then acidified while cooling with 30 ml of concentrated hydrochloric acid to pH 2 and the precipitated white solid is collected, washed with 150 ml of cold water, and dried *in vacuo* yielding 106.2 g of material, melting point 139—143°C. Crystallization from 300 ml of ethyl acetate yields 98.6 g of the title compound, melting point 142—145°C.

B) *(R)-4-Hydroxy-1-[(4-methylphenyl)sulfonyl]-L-proline, methyl ester*

The above N-tosyl-L-proline (24.25 g, 0.085 mole) is esterified with diazomethane in methanolether as described in *The Journal of the American Chemical Society, 79*, 191 (1957) yielding 25.3 g of product, melting point 101—104°C.

C) *(R)-1-[(4-Methylphenyl)sulfonyl]-4-[[(4-methylphenyl)sulfonyl]oxy]-L-proline, methyl ester*

A stirred solution of 23.95 g (0.08 mole) of the above *trans*-4-hydroxy-L-proline derivative in 50 ml of dry pyridine is treated dropwise at −5°C to −8°C with a solution of 17.16 g of *p*-toluenesulfonyl chloride (0.09 mole) in 20 ml of pyridine. The solution is stored in the cold room for 72 hours, added with stirring to 425 ml of ice-cold 2N hydrochloric acie, and the precipitated gum extracted with 200 ml of chloroform. The aqueous phase is extracted with additional chloroform (three 125 ml portions). The combined organic layers are dried ($MgSO_4$) and the solvent evaporated to give a viscous oil. The oil is dissolved in ethyl acetate (125 ml) and the product crystallized with cooling and rubbing to yield 26.8 g of *trans*-N,O-ditosylhydroxy-L-proline methyl ester, melting point 92—94°C.

D) *(S)-1-[(4-Methylphenyl)sulfonyl]-4-azido-L-proline, methyl ester*

A stirred solution of 22.68 g (0.05 moles) of the above *trans*-N,O-ditosylhydroxy-L-proline, methyl ester is dissolved in 150 ml of dimethylformamide, and 3.6 g (0.055 mole) of sodium azide in 17 ml of water is added. The reaction mixture is heated at 70°C for 5 hours. It is cooled, and poured into a mixture of saturated brine (1000 ml) and water (200 ml), and extracted with ether (three 250 ml portions). The combined ether extracts are washed with saturated brine and dried ($MgSO_4$). Removal of the ether and crystallization of the residue from ether/light petroleum ether yields 12.4 g of the title *cis*-azide ester as needles, melting point 69—70°C.

E) *(S)-4-Amino-1-[(4-methylphenyl)sulfonyl]-L-proline, methyl ester*

A solution of 9.73 g of the above *cis*-azide ester (0.03 mole) is dissolved in 100 ml of methanol and 1.0 g of 10% palladium on charcoal is added to the mixture. The mixture is hydrogenated for 6 hours at 50 psi. (The reaction bottle is flushed twice with hydrogen to remove the nitrogen as it is formed). The mixture is then filtered through Celite and the methanol removed *in vacuo* yielding 8.94 g of the amino product as a viscous oil. $[\alpha]_D^{26}$ −50° (c = , MeOH).

F) *(S)-4-(Benzoylamino)-1-[(4-methylphenyl)sulfonyl]-L-proline, methyl ester*

A stirred solution of 8.95 g (0.03 mole) of the above *cis*-amino ester and 2.4 g (0.031 mole) of triethylamine in 100 ml of chloroform is cooled to 0 to 5°C in an ice-bath and treated with 4.20 g (0.031 mole) of benzoyl chloride. The reaction is maintained under nitrogen and allowed to stir for about 16 hours at room temperature. The mixture is extracted with water (two 75 ml portions) followed by washing with 100 ml of a saturated salt solution. The chloroform is dried over anhydrous $MgSO_4$ and the chloroform removed *in vacuo* yielding a solid. Crystallization from 75% ethanol yields 10.46 g of the title compound, melting point 167—169°C.

G) *(S)-4-Benzoylamino-1-[(4-methylphenyl)sulfonyl]-L-proline*

The above ester (10.0 g, 0.0248 mole) is dissolved in 120 ml of methanol, treated dropwise at 0°C with 37.5 ml (0.0372 mole) of 2N sodium hydroxide kept at 0°C for 1 hour, and at room temperature for about 16 hours. After removing about 1/2 of the solvent on a rotary evaporator, the solution is cooled and acidified with 6N HCl to pH 2, and extracted with four 150 ml portions of ethyl acetate. The combined extracts are washed with 100 ml of a saturated salt solution dried ($MgSO_4$) and the solvent evaporated to give 7.18 g of the title compound, melting point 120—125°C (sintering at 118°C).

H) *(S)-4-(Benzoylamino)-L-proline, hydrobromide (1:1)*

The above N-tosyl acid (7.0 g, 0.017 mole) and phenol (3.2 g, 0.035 mole) are treated with 28 ml of hydrogen bromide in acetic acid (30—32%), stoppered loosely, and stirred for 18 hours. Ether

(300 ml) is added with swirling to the mixture and when the crystalline product has settled, the etheral liquor is decanted and the material is washed with 300 ml of fresh ether by decantation. The product is heated on the steam bath with 80 ml of methyl ethyl ketone, cooled, filtered, washed with cold methyl ethyl ketone and with ether, and dried *in vacuo*, yielding 2.6 g of the title compound, melting point 184—188°C, *dec.*

I) *[1(S),4S]-1-[3-(Acetylthio)-2-methyl-1-oxopropyl]-4-(benzoylamino)-L-proline*

The above amino acid hydrobromide salt (2.6 g, 0.0078 mole) and 1.6 g of (0.0086 mole) of D-3-(acetylthio)-2-methylpropionyl chloride in 5 ml of ether are reacted in 50 ml of water in the presence of $Na_2CO_3$. Initially, 2.4 ml of 25% $Na_2CO_3$ solution is required to obtain a pH of 8.0. Approximately 14 ml of 25% $Na_2CO_3$ (w/v) is consumed to stablize the pH of 8.0—8.2 during the acylation (time approx. 45 minutes). The mixture is allowed to warm to room temperature and stirred for 1—1/2 hours under argon. The mixture is worked up after an additional 1 hour by washing with 25 ml of ethyl acetate which is discarded, layered over with 25 ml of ethyl acetate, cooled, stirred, acidified carefully with 1:1 HCl to pH 2.0, saturated with salt and separated. The aqueous phase is extracted with three 25 ml portions of ethyl acetate, the combined organic layers are dried ($MgSO_4$) and the solvent removed *in vacuo*. The product is obtained as a syrupy oil, yield 2.30 g, which slowly begins to crystallize. The oil is treated in 30 ml of ethyl acetate with 1.5 g of dicyclohexylamine to give 2.18 g of the dicyclohexylamine salt of the title compound, melting point 160—163°C, $[\alpha]^{26}$—51°C (c = 1; methanol). Acidification of the dicyclohexylamine salt (2.18 g) with 26 ml of 10% $KHSO_4$ and extraction with 40 ml of ethyl acetate (four times) yields 1.1 g of the title compound, melting point 82—84°C, $[\alpha]_D^{26}$ −81° (c = 1; $CHCl_3$).

J) *[1(S),4S]-4-(Benzoylamino)-1-(3-mercapto-2-methyl-1-oxopropyl)-L-proline*

Argon is passed through a cold solution of 1.5 ml of concentrated ammonium hydroxide in 8.0 ml of water for 0.25 hours which is then added while cooling and under a blanket of argon to 1.0 g (0.0026 mole) of the above S-acetyl compound. The mixture is swirled in an ice-bath until a solution is obtained. Stirring under argon is continued at room temperature for an additional 2 hours, then the solution is extracted with 15 ml of ethyl acetate (this and subsequent operations are carried out as much as possible under an argon atmosphere). The aqueous layer is cooled, stirred, layered over with 15 ml of ethyl acetate and acidified portionwise with approximately 4.2 ml of 6N HCl. The layers are separated, the aqueous phase extracted with three 15 ml portions of ethyl acetate, the combined ethyl acetate layers dried ($MgSO_4$) and the solvent evaporated to give 0.46 g of the title compound after drying *in vacuo*, melting point 150—152°C, $[\alpha]_D^{26}$ −44° (c = 1; EtOH).

## Example 2

[1(S),4S]-1-[3-(Acetylthio)-2-methyl-1-oxopropyl]-4-(1,3-dihydro-1,3-dioxo-2H-isoindol-2-yl)-L-proline

A) *(S)-4-(1,3-Dihydro-1,3-dioxo-2H-isoindol-2-yl)-1-[(phenylmethoxy)carbonyl]-L-proline, phenylmethyl ester*

To a solution of 7.0 g of N-(benzyloxycarbonyl)-4-*trans*-hydroxy-L-proline, benzyl ester (described in Can. J. Biochem. & Phy., *37*, 583 (1959)) 4.34 g of phthalimide, and 7.73 g of triphenylphosphine in 200 ml of dry (sieve) tetrahydrofuran is added 5.13 g of diethyl azodicarboxylate over 1 hour at room temperature. Thin layer chromatography (silica gel-ether) shows consumption of starting material is complete in less than two hours. The solvent is removed under vacuum and the residue diluted with 200 ml of ether. The resulting precipitate is filtered off and the filtrate is concentrated to about 40 ml. This material is flash chromatographed on silica gel with ether:pentane (1:1) to yield a total of 9.15 g of residue. The product is isolated in two fractions A (first portion eluted) and AA (second portion eluted). Both fractions are taken up in ether and allowed to stand. The resulting cyrstals are filtered off to yield the final product $A^1$ and $AA^1$. $A^1$ melts at 89—90°C while $AA^1$ melts at 117—119°C, but on standing for several days the melting point changes to 89—90°C, $[\alpha]_D^{25}$ −47.8° (a = 0.5%; $CHCl_3$). Both fractions are identical chromatographically and spectrally (NMR) and are combined for further use.

B) *[1(S,4S]-1-[3-(Acetylthio)-2-methyl-1-oxopropyl]-4-(1,3-dihydro-1,3-dioxo-2-H-isoindol-2-yl)-L-proline*

A solution of 5.0 g of (S) - 4 - (1,3 - dihydro - 1,3 - dioxo - 2H - isoindol - 2 - yl) - 1 - [(phenylmethoxy)carbonyl] - L - proline, phenylmethyl ester in 200 ml of ethyl acetate and 100 ml of ethanol is hydrogenated with 2.0 g of 10% palladium on charcoal at room temperature and 40 psi. After 2 days the mixture is filtered to yield a mixture of the amino acid product and catalyst as the filter cake.

The mixture of catalyst and amino acid is suspended in 75 ml of dry pyridine and treated with 3 g of (S) - 3 - (acetylthio) - 2 - methylpropionyl chloride at room temperature. After 1 hour another 2 g of acid chloride is added. The mixture is stirred for 2 hours and filtered. The filtrate is evaporated to dryness and the residue partitioned between 1N HCl and ethyl acetate. The ethyl acetate solution is washed with water and saturated NaCl, dried ($Na_2SO_4$) and the solvents are removed under vacuum.

The residue is chromatographed on silica gel with 7:1 toluene-acetic acid. The fraction with an $R_f$ of 0.1 (tlc) is collected and evaporated to dryness. The residue is taken up in ethyl acetate and filtered. The filtrate is again evaporated to dryness to yield 2.8 g of an oil. The oil is taken up in ether and allowed to stand for about 16 hours. The resulting crystals are filtered off and washed with ethyl acetate to yield the product, melting point 185—188°C; $[\alpha]_D^{25}$—95.3° (c= 1%; $CHCl_3$).

Example 3

[1(S),4S]-4-Amino-1-(3-mercapto-2-methyl-1-oxopropyl)-L-proline

To 0.8 g of [1(S),4S] - 1 - [3 - (acetylthio) - 2 - methyl - 1 - oxopropyl] - 4 - (1,3 - dihydro - 1,3 - dioxo - 2H - isoindol - 2 - yl) - L - proline suspended in 20 ml of methanol is added 36 drops of anhydrous hydrazine. After 30 minutes the mixture is concentrated to 5 ml and placed on a column of DEAE Sephadex which is eluted with a 0.005M to 0.5 molar gradient of aqueous ammonium carbonate. The fractions giving a positive sulfhydryl test (nitroprusside solution) are combined and lyophilized to yield the partially purified product. further purification is achieved by chromatography on silica gel and elution with butanol:acetic acid:water (4:1:1).

Examples 4—17

Following the procedure of Example 2, but substituting the compound listed in Column I for phthalimide, yields the compound listed in Column II.

| | Column I | Column II |
|---|---|---|
| 4 | hexahydrophthalimide | [1(S),4S]-1-[3-(acetylthio)-2-methyl-1-oxopropyl]-4-(hexahydro-2H-isoindol-2-yl-1,3-dione)-L-proline |
| 5 | 1,8-naphthalenecarboximide | [1(S),4S]-1-[3-(acetylthio)-2-methyl-1-oxopropyl]-4-(1,3-dihydro-1,3-dioxo-2H-benz[de]isoquinolin-2-yl)-L-proline |
| 6 | saccharin | (1(S),4S]-1-[3-(acetylthio)-2-methyl-1-oxopropyl]-4-(1,1,3-trioxo-1,2-benzisothiazol-2(3H)-yl)-L-proline |
| 7 | succinimide | [1(S),4S]-1-[3-(acetylthio)-2-methyl-1-oxopropyl]-4-(1-pyrrolidinyl-2,5-dione-L-proline |
| 8 | glutarimide | [1(S),4S]-1-[3-(acetylthio)-2-methyl-1-oxopropyl]-4-(1-piperidinyl)-2,6-dione-L-proline |
| 9 | maleimide | [1(S),4S]-1-[3-(acetylthio)-2-methyl-1-oxopropyl]-4-(2,5-dihydro-2,5-dioxo-1H-pyrrol-1-yl)-L-proline |
| 10 | diacetamide | [1(S),4S]-1-[3-(acetylthio)-2-methyl-1-oxopropyl]-4-diacetamido-L-proline |
| 11 | dibenzamide | [1(S),4S]-1-[3-(acetylthio)-2-methyl-1-oxopropyl]-4-dibenzamido-L-proline |
| 12 | N-acetylbenzamide | [1(S),4S]-1-[3-(acetylthio)-2-methyl-1-oxopropyl]-4-(N-acetylbenzamido)-L-proline |
| 13 | diphenacetamide | [1-(S),4S]-1-[3-(acetylthio)-2-methyl-1-oxopropyl]-4-diphenacetamido-L-proline |
| 14 | dicinnamoylamine | [1(S),4S]-1-[3-(acetylthio)-2-methyl-1-oxopropyl]-4-dicinnamoylamino-L-proline |
| 15 | dimethylsulfonamide | [1(S),4S]-1-[3-(acetylthio)-2-methyl-1-oxopropyl]-4-dimethylsulfonamido-L-proline |
| 16 | dibenzenesulfonamide | [1(S),4S]-1-[3-(acetylthio)-2-methyl-1-oxopropyl]-4-dibenzenesulfonamido-L-proline |
| 17 | N-(benzenesulfonyl)-acetamide | [1(S),4S]-1-[3-(acetylthio)-2-methyl-1-oxopropyl]-4-(N-(benzenesulfonyl)acetamido)-L-proline |

Examples 18—29

Following the procedure of Example 1, but substituting the compound listed in Column I for benzoyl chloride in part F, yields the compound listed in Column II.

| | Column I | Column II |
|---|---|---|
| 18 | 4-fluorobenzoyl chloride | [1(S),4S]-4-(4-fluorobenzoylamino)-1-(3-mercapto-2-methyl-1-oxopropyl)-L-proline |
| 19 | 2,6-dichlorobenzoyl chloride | [1(S),4S]-4-(2,6-dichlorobenzoylamino)-1-(3-mercapto-2-methyl-1-oxopropyl)-L-proline |
| 20 | 2,4,6-trimethylbenzoyl chloride | [1(S),4S]-4-(2,4,6-trimethylbenzoylamino)-1-(3-mercapto-2-methyl-1-oxopropyl)-L-proline |
| 21 | 3,4-dimethoxybenzoyl chloride | [1(S,4S]-4-(3,4-dimethoxybenzoylamino)-1-(3-mercapto-2-methyl-1-oxopropyl)-L-proline |
| 22 | 4-ethylthiobenzoyl chloride | [1(S),4S]-4-(4-ethylthiobenzoylamino)-1-(3-mercapto-2-methyl-1-oxopropyl)-L-proline |
| 23 | 2-hydroxybenzoyl chloride | [1(S),4S]-4-(2-hydroxybenzoylamino)-1-(3-mercapto-2-methyl-1-oxopropyl)-L-proline |
| 24 | 4-acetylbenzoyl chloride | [1(S),4S]-4-(4-acetylbenzoylamino)-1-(3-mercapto-2-methyl-1-oxopropyl)-L-proline |
| 25 | 4-nitrobenzoyl chloride | [1(S),4S]-4-(4-nitrobenzoylamino)-1-(3-mercapto-2-methyl-1-oxopropyl)-L-proline |
| 26 | 3-fluoro-4-methoxy-benzoyl chloride | [1(S),4S]-4-(3-fluoro-4-methoxybenzoylamino)-1-(3-mercapto-2-methyl-1-oxopropyl)-L-proline |
| 27 | 4-dimethylaminobenzoyl chloride | [1(S),4S]-4-(4-dimethylaminobenzoylamino)-1-(3-mercapto-2-methyl-1-oxopropyl)-L-proline |
| 28 | 4-phenylbenzoyl chloride | [1(S),4S]-4-(4-phenylbenzoylamino)-1-(3-mercapto-2-methyl-1-oxopropyl)-L-proline |
| 29 | 3-trifluoromethylbenzoyl chloride | [1(S),4S]-4-(3-trifluoromethylbenzoylamino)-1-(3-mercapto-2-methyl-1-oxopropyl)-L-proline |

Example 30

[1(S),4S]-1-(3-Mercapto-2-methyl-1-oxopropyl)-4-(1-piperidinyl)-L-proline, hydrochloride

A) *[4S]-1-[(Phenylmethoxy)carbonyl]-4-(1-piperidinyl)-L-proline, methyl ester*

A mixture of 15.0 g of 1 - [(phenylmethoxy)carbonyl] - *trans* - 4 - tosyloxy - L - proline, methyl ester (J.A.C.S., *79*, 191 (1957)) and 75 ml of piperidine is stirred at room temperature for 24 hours. The excess piperidine is removed under reduced pressure and the residue is dissolved in 50 ml of chloroform. The latter solution is extracted twice with 10 ml portions of water, dried ($MgSO_4$), filtered and the solvent evaporated to give the title compound.

B) *[4S]-4-(1-Piperidinyl)-L-proline, methyl ester*

A solution of 10.0 g of [4S] - 1 - [(phenylmethoxy)carbonyl] - 4 - (1 - piperidinyl) - L - proline, methyl ester in 100 ml of methanol is treated with a suspension of 2 g of 5% palladium on charcoal in 10 ml of water and placed under 3 atmospheres of hydrogen. After an equivalent quantity of hydrogen is consumed, the suspension is filtered and the solvent evaporated to give the title compound.

C) *[1(S),4S]-1-[3-(Acetylthio)-2-methyl-1-oxopropyl]-4-(1-piperidinyl)-L-proline, methyl ester, hydrochloride*

A solution of 7.0 g of [4S] - 4 - (1 - piperidinyl) - L - proline, methyl ester in 100 ml of chloroform is stirred and maintained at 20—25°C during the addition of an equivalent quantity of (S)-3-(acetylthio)-2-methylpropionyl chloride. After stirring for 4 hours at room temperature, the solvent is removed yielding the title compound.

D) *[1(S),4S]-1-(3-Mercapto-2-methyl-1-oxopropyl)-4-(1-piperidinyl)-L-proline, hydrochloride*

A solution of 5.0 g of [1(S),4S] - 1 - [3 - (acetylthio) - 2 - methyl - 1 - oxopropyl] - 4 - (1 - piperidinyl) - L - proline, methyl ester, hydrochloride in 50 ml of dioxane is treated with 3.0 ml of a saturated solution of hydrogen chloride in methanol, and the resulting solution is allowed to stand for 12 hours at 0—10°C. The solvent is evaporated to give the title compound.

Examples 31—44

Following the procedure of Example 30, but substituting the compound listed in Column I for piperidine, yields the compound listed in Column II.

| | Column I | Column II |
|---|---|---|
| 31 | pyrrolidine | [1(S),4S]-1-(3-mercapto-2-methyl-1-oxopropyl)-4-(1-pyrrolidinyl)-L-proline, hydrochloride |
| 32 | morpholine | [1(S),4S]-1-(3-mercapto-2-methyl-1-oxopropyl)-4-(4-morpholinyl)-L-proline, hydrochloride |
| 33 | 4-methylpiperazine | [1(S),4S]-1-(3-mercapto-2-methyl-1-oxopropyl)-4-(4-methyl-1-piperazinyl)-L-proline, hydrochloride |
| 34 | 4-phenylpiperazine | [1(S),4S]-1-(3-mercapto-2-methyl-1-oxopropyl)-4-(4-phenyl-1-piperazinyl)-L-proline, hydrochloride |
| 35 | imidazole | [1(S),4S]-1-(3-mercapto-2-methyl-1-oxopropyl)-4-(1-imidazolyl)-L-proline, hydrochloride |
| 36 | diethylamine | [1(S),4S]-1-(3-mercapto-2-methyl-1-oxopropyl)-4-(diethylamino)-L-proline, hydrochloride |
| 37 | cyclohexylamine | [1(S),4S]-1-(3-mercapto-2-methyl-1-oxopropyl)-4-(cyclohexylamino)-L-proline, hydrochloride |
| 38 | 1-adamantylamine | [1(S),4S]-1-(3-mercapto-2-methyl-1-oxopropyl)-4-(1-adamantylamino)-L-proline, hydrochloride |
| 39 | N-methyl-1-adamantylamine | [1(S),4S]-1-(3-mercapto-2-methyl-1-oxopropyl)-4-(N-methyl-1-adamantylamino)-L-proline, hydrochloride |
| 40 | *t*-butylamine | [1(S),4S]-1-(3-mercapto-2-methyl-1-oxopropyl)-4-(*t*-butylamino)-L-proline, hydrochloride |
| 41 | aniline | [1(S),4S]-1-(3-mercapto-2-methyl-1-oxopropyl)-4-(phenylamino)-L-proline, hydrochloride |
| 42 | *p*-chloroaniline | [1(S),4S]-1-(3-mercapto-2-methyl-1-oxopropyl)-4-(4-chlorophenylamino)-L-proline, hydrochloride |
| 43 | benzylamine | [1(S),4S]-1-(3-mercapto-2-methyl-1-oxopropyl-4-(benzylamino)-L-proline, hydrochloride |
| 44 | phenethylamine | [1(S),4S]-1-(3-mercapto-2-methyl-1-oxopropyl)-4-(phenylethylamino)-L-proline, hydrochloride |

Examples 45—48

Following the procedure of Example 1, but substituting the compound listed in Column I for D - 3 - (acetylthio) - 2 - methylpropionyl chloride, yields the compound listed in Column II.

| Column I | | Column II |
|---|---|---|
| 45 | 3-(acetylthio)-2-(trifluoromethyl)propionyl chloride | (4S)-4-(benzoylamino)-1-(3-mercapto-2-trifluoromethyl-1-oxopropyl)-L-proline |
| 46 | 4-(acetylthio)butyryl chloride | (4S)-4-(benzoylamino)-1-(4-mercapto-1-oxobutyl)-L-proline |
| 47 | 2-(acetylthio)acetyl chloride | (4S)-4-(benzoylamino)-1-(3-mercapto-1-oxoethyl)-L-proline |
| 48 | 3-(acetylthio)-2-(methylthio)propionyl chloride | (4S)-4-(benzoylamino)-1-[3-mercapto-2-(methylthio)-1-oxopropyl)-L-proline |

Example 49

[1(S),4S]-4-(Benzoylamino)-1-(3-mercapto-2-methyl-1-oxopropyl)-L-proline, sodium salt

A suspension of 5.0 g of [1(S),4S] - 4 - (benzoylamino) - 1 - (3 - mercapto - 2 - methyl - 1 - oxopropyl) - L - proline is suspended in 15 ml of water and treated with 1 equivalent of sodium bicarbonate. The solution is evaporated under reduced pressure to give the title compound.

Example 50

[1(S),4S]-4-(1-Homopiperidinyl)-1-(3-mercapto-2-methyl-1-oxopropyl)-L-proline

Following the procedure of Example 30, but substituting homopiperidine for piperidine in part A, yields the title compound.

Example 51

[1(S),4S]-(3-Mercapto-2-methyl-1-oxopropyl)-4-(1-naphthoylamino)-L-proline

Following the procedure of Example 1, but substituting 1-naphthoyl chloride for benzoyl chloride in part F, yields the title compound.

Example 52

[1(S),4S]-(3-Mercapto-2-methyl-1-oxopropyl)-4-(2-naphthoylamino)-L-proline

Following the procedure of Example 1, but substituting 2-naphthoyl chloride for benzoyl chloride in part F, yields the title compound.

Example 53

[1(S),1′(S), 4R, 4′R]-1,1′-[Dithiobis(2-methyl-1-oxopropane-3,1-diyl)bis[4-benzoylamino-L-proline

A solution of the product from Example 1 is dissolved in ethanol, stirred and treated with a solution of one equivalent of iodine in ethanol. The pH of the solution is maintained at 6—7 by the addition of N-sodium hydroxide solution. The solvent is evaporated and the residue extracted with ethyl acetate. After drying over $MgSO_4$, the solution is filtered and the solvent evaporated to give the title compound.

Example 54

[1(S)]-4-(Benzoylamino)-1-(3-mercapto-2-methyl-1-oxopropyl)-DL-pipecolinic acid

Following the procedure of Example 1, but substituting DL-4-hydroxypipecolinic acid for *trans*-4-hydroxy-L-proline in part A, the title compound is obtained.

Example 55

[1(S),4S]-4-Azido-1-[3-(benzoylthio)-2-methyl-1-oxopropyl]-L-proline, methyl ester

A) *[1(S),4R]-1-[3-(benzoylthio)-2-methyl-1-oxopropyl]-4-hydroxy-L-proline, methyl ester*

*trans*-4-Hydroxy-L-proline, methyl ester (0.02 mole) is dissolved in 75 ml of dichloromethane and cooled to 5°C in an ice-bath under argon. 3-(Benzoylthio)-2-methylpropanoic acid (4.49 g, 0.02 mole) and N,N′-dicyclohexylcarbodiimide (4.13 g, 0.02 mole) in 25 ml of dichloromethane are then added. After 0.5 hours the ice-bath is removed and the reaction is allowed to warm to room temperature and stir for about 16 hours. The mixture is filtered and the filtrate concentrated *in vacuo* yielding an oily residue. The residue is taken up in either (30 ml) and filtered to remove additional solid, redissolved in 125 ml of dichloromethane and washed with 50 ml each of 5% $KHSO_4$, saturated $NaHCO_3$, brine, and

dried ($MgSO_4$). The residue after evaporation is preabsorbed on 120 ml of silica gel and the material chromatographed on silica gel (1000 ml), eluting with ethyl acetate, methylene chloride (1:4) to give 6.1 g of the title compound.

B) *[1(S),4S]-4-Azido-1-[3-(benzoylthio)-2-methyl-1-oxopropyl]-L-proline, methyl ester*

Diethylazodicarboxylate (1.04 g, 0.006 mole) in acetonitrile (5 ml) is added to a mixture of [1(S),4R] - 1 - [3 - (benzoylthio) - 2 - methyl - 1 - oxopropyl] - 4 - hydroxy - L - proline, methyl ester (1.05 g, 0.003 mole), triphenylphosphine (1.58 g, 0.006 mole) and 12.5 ml of 5.4% hydrazoic acid in benzene (0.015 mole, 5 equivalents) in 40 ml of dry acetonitrile over 0.5 hour with stirring. After 6 hours, thin layer chromatography (tlc) shows the starting proline derivative to be still present, so additional triphenylphosphine (0.40 g, 0.0015 mole) diethylazodicarboxylate (0.26 g, 0.0015 mole) and hydrazoic acid solution in benzene (4.0 ml, 0.0075 mole) are added and the mixture is allowed to stir for 6 hours. The solvents are removed by evaporation *in vacuo* yielding a semi-solid residue. Trituration with ether and filtration removes most of the solid. The crude product is preadsorbed on 25 ml of silica gel and the material is then flash chromatographed on silica gel (250 ml), eluting with ether:hexane (1:1) to yield 0.71 g of pure product as an oil (tlc $R_f = 0.48$, ether). IR shows absence of hydroxy group (3310 $cm^{-1}$) and presence of azide (2120 $cm^{-1}$).

Example 56

[1(S),4S]-4-Azido-1-(3-mercapto-2-methyl-1-oxopropyl)-L-proline

[1(S),4S]-4-Azido-1-[3-(benzoyltho)-2-methyl-1-oxopropyl]-L-proline, methyl ester (1.0 g, 0.00266 mole) is dissolved in 25 ml of absolute methanol and the mixture is purged three times with argon. Sodium hydroxide (5.5 ml, 1.0 N) is added under argon (total exclusion of air is necessary) and the mixture is stirred for 24 hours. The mixture is acidified with 1.0 N hydrochloric acid to pH 5 and extracted with three 30 ml portions of ethyl acetate. The extracts are combined, dried ($MgSO_4$) and evaporated yielding 0.65 g oily syrup. The crude material is preadsorbed on 25 ml of silica gel and flash chromatographed on silica gel (250 ml), eluting with benzene/acetic acid (4:1) to yield 0.468 g of the title compound as a heavy oil.

A portion of the above oil (0.100 g, 0.0039 mole) is dissolved in 10 ml of ethyl acetate and 1 equivalent of adamantene amine is added with stirring. The white precipitate is collected and washed with cold ethyl acetate to yield the adamantane amine salt of the title compound, melting point 168—172°C.

*Analysis** calc'd for $C_{20}H_{33}N_5SO_{3.5}$, MW 431.57 C, 55.66; H, 7.71; N, 16.33; S, 7.43 Found: C, 55.56; H, 7.89; N, 16.49; S, 7.59; SH, 99.6%.

*(1:1) Adamantane amine salt + 1/4 mole of ethyl acetate.

Examples 57—61

Following the procedure of Example 55, but substituting the compound listed in column I for *trans*-4-hydroxy-L-proline methyl ester and the compound listed in column II for 3-(benzoylthio)-2-methylpropanoic acid, yields the compound listed in column III.

| | Column I | Column II | Column III |
|---|---|---|---|
| 57 | *trans*-4-hydroxy pipecolic acid, methyl ester | 3-(acetylthio)-2-methylpropanoic acid | [1(S),4S]-1-[3-(acetylthio)-2-methyl-1-oxopropyl]-4-azidopipecolic acid, methyl ester |
| 58. | *trans*-4-hydroxy-L-proline, methyl ester | 3-(acetylthio)-2-(trifluoromethyl) propanoic acid | [1(S),4S]-1-[3-(acetylthio)-1-oxo-2-(trifluoromethyl)-propyl]-4-azido-L-proline, methyl ester |
| 59. | *trans*-4-hydroxy-L-pipecolic acid, benzyl ester | 3-(acetylthio)-2-(methylthio)propanoic acid | [1(S),4S]-1-[3-(acetylthio)-2-(methylthio)-1-oxopropyl]-4-azido-L-pipecolic acid, benzyl ester |
| 60. | *trans*-4-hydroxy-L-proline, benzyl ester | 3-(benzoylthio)-propanoic acid | [1(S),4S]-4-azido-1-[3-benzoyl-thio)-1-oxopropyl]-L-proline, benzyl ester |
| 61. | *trans*-4-hydroxy-L-proline, methyl ester | 4-(acetylthio)-butyric acid | [1(S),4S]-1-[4-(acetylthio)-1-oxobutyl]-4-azido-L-proline, methyl ester |

**Claims**

1. A compound having the formula

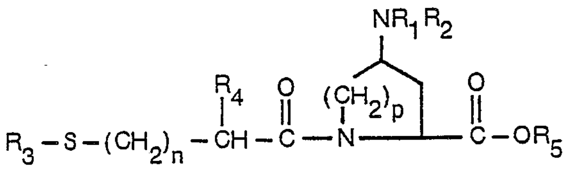

or a salt thereof, or symmetrical dimer thereof, *i.e.,* compound having the formula

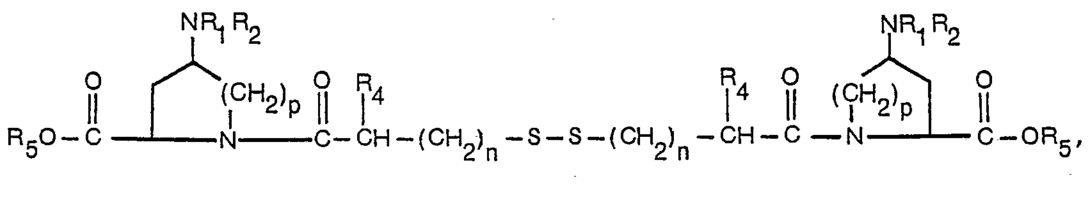

wherein —$NR_1$—$R_2$ is azido or $R_1$ and $R_2$ are the same or different and are hydrogen, alkyl, cycloalkyl, 1-adamantyl, aryl, arylalkyl, alkylcarbonyl, arylcarbonyl, arylalkylcarbonyl, alkylsulfonyl, arylsulfonyl, or arylvinylcarbonyl, or together with the nitrogen atom to which they are attached $R_1$ and $R_2$ are 1-pyrrolidinyl, 1-piperidinyl, 1-homopiperidinyl, 4-morpholinyl, 4-alkyl-1-piperazinyl, 4-aryl-1-piperazinyl, 1-imidazolyl, 1-pyrrolidinyl-2,5-dione, 3-alkyl-1-pyrrolidinyl-2,5-dione, 3-aryl-1-pyrrolidinyl-2,5-dione, 1-piperidinyl-2,6-dione, 3-alkyl-1-piperidinyl-2,6-dione, 3-aryl-1-piperidinyl-2,6-dione, 2H-isoindol-2-yl-1,3-dione, hexahydro-2H-isoindol-2-yl-1,3-dione, 2,5-dihydro-2,5-dioxo-1H-pyrrol-1-yl, 1,1,3-trioxo-1,2-benzisothiazol-2(3H)-yl, or 1,3-dihydro-1,3-dioxo-2H-benz[de]isoquinolin-2-yl;

$R_3$ is hydrogen, alkyl, aryl, arylalkyl, or a hydrolyzable acyl protecting group;

$R_4$ is hydrogen, alkyl, alkylthio, or trifluoromethyl;

n is 0, 1 or 2; and

p is 1 or 2;

$R_5$ is hydrogen, alkyl, or arylalkyl;

"aryl", either by itself or as part of a larger group, being phenyl, 1-naphthyl, 2-naphthyl or phenyl substituted with halogen, alkyl, alkoxy, alkylthio, hydroxy, alkanoyl, nitro, amino, dialkylamino, phenyl or trifluoromethyl;

"cycloalkyl", either by itself or as part of a larger group, referring to cycloalkyl groups having 3 to 7 carbon atoms;

"alkyl", either by itself or as part of a larger group, referring to groups having 1 to 8 carbon atoms;

"alkoxy" either by itself or as part of a larger group, referring to groups having 1 to 8 carbon atoms;

"halogen", either by itself or as part of a larger group, being fluorine, chlorine, bromine or iodine;

"alkanoyl", either by itself or as part of a larger group, referring to groups having 1 to 9 carbon atoms.

2. A compound in accordance with claim 1 wherein $R_3$ is hydrogen.

3. A compound in accordance with claim 1 wherein $R_3$ is a hydrolyzable protecting group selected from the group consisting of alkanoyl and arylcarbonyl as defined in claim 1.

4. A compound in accordance with claim 1 wherein $R_4$ is hydrogen.

5. A compound in accordance with claim 1 wherein $R_4$ is alkyl as defined in claim 1.

6. A compound in accordance with claim 1 wherein $R_4$ is alkylthio as defined in claim 1.

7. A compound in accordance with claim 1 wherein $R_4$ is trifluoromethyl.

8. A compound in accordance with claim 1 wherein n is 1.

9. A compound in accordance with claim 1 wherein n is 2.

10. A compound in accordance with claim 1 wherein p is 1.

11. A compound in accordance with claim 1 wherein p is 1.

12. A compound in accordance with claim 1 wherein $R_5$ is hydrogen.

13. A compound in accordance with claim 1 wherein n is 1, p is 1, $R_4$ is methyl, and $R_5$ is hydrogen.

14. The compound in accordance with claim 1, [1(S),4S] - 4 - (benzoylamino) - 1 - (3 - mercapto - 2 - methyl - 1 - oxopropyl) - L - proline.

15. The compound in accordance with claim 1, [1(S),4S] - 1 - [3 - acetylthio) - 2 - methyl - 1 - oxopropyl] - 4 - (1,3 - dihydro - 1,3 - dioxo - 2H - isoindol - 2 - yl) - L - proline.

16. A compound in accordance with claim 1 wherein —$NR_1R_2$ is azido.

17. A compound in accordance with claim 16 wherein $R_3$ is hydrogen.

18. A compound in accordance with claim 16 wherein n is 1, p is 1 and $R_2$ is methyl.

19. The compound in accordance with claim 1 [1(S),4S] - 4 - azido - 1 - [3 - (benzoylthio) - 2 - methyl - 1 - oxopropyl] - L - proline, methyl ester.

20. The compound in accordance with claim 1 [1(S),4S] - 4 - azido - 1 - (3 - mercapto - 2 - methyl - 1 - oxopropyl) - L - proline.

**Patentansprüche**

1. Eine Verbindung der Formel

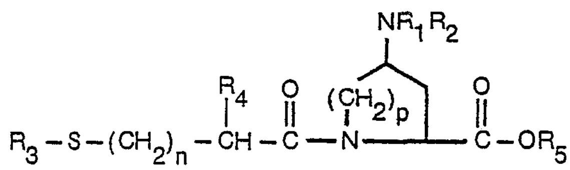

oder ihres Salzes oder ihres symmetrischen Dimeren, nämlich einer Verbindung der Formel

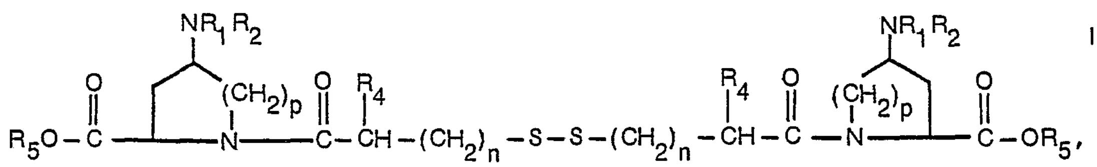

IX

wobei in den Formeln N—$NR_1R_2$ eine Azidogruppe bedeutet oder $R_1$ und $R_2$ gleich oder verschieden sind und ein Wasserstoffatom, einen Alkyl-, Cycloalkyl-, 1-Adamantyl-, Aryl-, Arylalkyl-, Alkylcarbonyl-, Arylcarbonyl-, Arylalkylcarbonyl-, Alkylsulfonyl-, Arylsulfonyl oder Arylvinylcarbonylrest bedeuten, oder $R_1$ und $R_2$ zusammen mit dem Stickstoffatom, mit dem sie verknüpft sind, die folgenden Reste darstellen: 1-Pyrrolidinyl-, 1-Piperidinyl-, 1-Homopiperidinyl-, 4-Morpholinyl-, 4-Alkyl-1-piperazinyl-, 4-Aryl-1-piperazinyl-, 1-Imidazolyl-, 1-Pyrrolidinyl-2,5-dion-, 3-Alkyl-1-pyrrolidinyl-2,5-dion-, 3-Aryl-1-pyrrolidinyl-2,5-dion-, 1-Piperidinyl-2,6-dion-, 3-Alkyl-1-piperidinyl-2,6-dion-, 3-Aryl-1-piperidinyl-2,6-dion-, 2H-Isoindol-2-yl-1,3-dion-, Hexahydro-2H-isoindol-2-yl-1,2-dion-, 2,5-Dihydro-2,5-dioxo-1H-pyrrol-1-yl-, 1,1,1-Trioxo-1,2-benzoisothiazol-2(3H)-yl- oder 1,3-Dihydro-1,3-dioxo-2H-benz[de]-isochinolin-2-yl-;

$R_3$ ein Wasserstoffatom, einen Alkyl-, Aryl- oder Arylalkylrest oder eine Hydrolysierbare Acylschutzgruppe bedeutet,

$R_4$ ein Wasserstoffatom-, einen Alkyl-, Alkylthio- oder Trifluormethylrest bedeutet;

$R_5$ ein Wasserstoffatom, einen Alkyl- oder Arylalkylrest bedeutet,

n 0, 1 oder 2 ist und

p 1 oder 2 ist und

der Ausdruck "Aryl" entweder selbst oder als Teil einer größeren Gruppe die folgende Bedeutung hat: Phenyl-, 1-Naphthyl-, 2-Naphthyl- oder Phenyl-, substituiert mit folgenden Resten: Halogen-, Alkyl-, Alkoxy-, Alkylthio-, Hydroxy-, Alkanoyl-, Nitro-, Amino-, Dialkylamino-, Phenyl- oder Trifluormethyl;

des Ausdruck "Cycloalkyl" entweder selbst oder als Teil einer größeren Gruppe eine Cycloalkylgruppe mit 3 bis 7 Kohlenstoffatomen bedeutet;

der Ausdruck "Alkyl" entweder selbst oder als Teil einer größeren Gruppe eine Gruppe mit 1 bis 8 Kohlenstoffatomen bedeutet;

der Ausdruck "Alkoxy" entweder selbst oder als Teil einer größeren Gruppe eine Gruppe mit 1 bis 8 Kohlenstoffatomen bedeutet;

der Ausdruck "Halogen" entweder selbst oder als Teil einer größeren Gruppe ein Fluor-, Chlor-, Brom- oder Jodatom bedeutet und

der Ausdruck "Alkanoyl" entweder selbst oder als Teil einer größeren Gruppe eine Gruppe mit 1 bis 9 Kohlenstoffatomen bedeutet.

2. Verbindung nach Anspruch 1, in der $R_3$ ein Wasserstoffatom bedeutet.

3. Verbindung nach Anspruch 1, in der $R_3$ eine hydrolysierbare Schutzgruppe aus der Gruppe Alkanoyl- und Arylcarbonyl- gemäß Anspruch 1 bedeutet.

4. Verbindung nach Anspruch 1, in der $R_4$ ein Wasserstoffatom bedeutet.

5. Verbindung nach Anspruch 1, in der $R_4$ eine Alkylgruppe gemäß Anspruch 1 bedeutet.

6. Verbindung nach Anspruch 1, in der $R_4$ eine Alkylthiogruppe nach Anspruch 1 bedeutet.

7. Verbindung nach Anspruch 1, in der $R_4$ eine Trifluormethylgruppe bedeutet.

8. Verbindung nach Anspruch 1, in der n den Wert 1 hat.

9. Verbindung nach Anspruch 1, in der n den Wert 2 hat.

10. Verbindung nach Anspruch 1, in der p den Wert 1 hat.

11. Verbindung nach Anspruch 1, in der p den Wert 2 hat.

12. Verbindung nach Anspruch 1, in der $R_5$ ein Wasserstoffatom bedeutet.

13. Verbindung nach Anspruch 1, in der n den Wert 1 und p den Wert 1 hat, $R_4$ eine Methylgruppe bedeutet und $R_5$ ein Wasserstoffatom darstellt.

14. Verbindung nach Anspruch 1, nämlich [1(S),4S] - 4 - (Benzoylamino) - 1 - (3 - mercapto - 2 - methyl - 1 - oxopropyl) - L - prolin.

15. Verbindung nach Anspruch 1, nämlich [1(S),4S] - 1 - [3 - Acetylthio) - 2 - methyl - 1 - oxopropyl] - 4 - (1,3 - dihydro - 1,3 - dioxo - 2H - isoindol - 2 - yl) - L - prolin.

16. Verbindung nach Anspruch 1, in der —$NR_1R_2$ eine Azidogruppe bedeutet.

17. Verbindung nach Anspruch 16, in der $R_3$ ein Wasserstoffatom bedeutet.

18. Verbindung nach Anspruch 16, in der n den Wert 1 und p den Wert 1 hat und $R_2$ eine Methylgruppe bedeutet.

19. Verbindung nach Anspruch 1, nämlich [1(S),4S] - 4 - Azido - 1 - [3 - (benzoylthio) - 2 - methyl - 1 - oxopropyl] - L - prolin - methylester

20. Verbindung nach Anspruch 1, nämlich [1(S),4S] - 4 - Azido - 1 - (3 - mercapto - 2 - methyl - 1 - oxopropyl) - L - prolin.

**Revendications**

1. Composé de formule

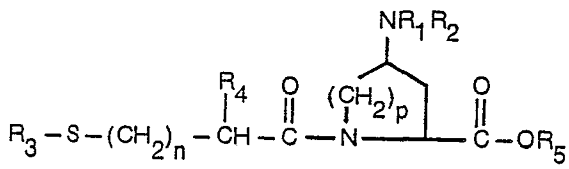

ou un sel de ce composé, ou un dimère symétrique de ce composé, c'est-à-dire un composé ayant pour formule

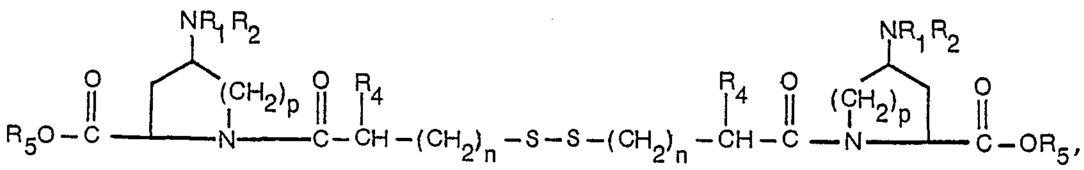

dans laquelle —$NR_1$—$R_2$ est un groupement azide ou bien $R_1$ et $R_2$ sont identiques ou différents et sont chacun un atome d'hydrogène ou un radical alkyle, cycloalkyle, 1-adamantyle, aryle, arylakyle, alkylcarbonyle, arylcarbonyle, arylalkylcarbonyle, alkylsulfonyle, arylsulfonyle ou arylvinylcarbonyle, ou bien, avec l'atome d'azote auquel ils sont fixés, $R_1$ et $R_2$ forment un radical 1-pyrrolidinyle, 1-pipéridinyle, 1-homopipéridinyle, 4-morpholinyle, 4-alkyl-1-pipérazinyle, 4-aryl-1-pipérazinyle, 1-imidazolyle, 1-pyrrolidinyl-2,5-dione, 3-alkyl-1-pyrrolidinyl-2,5-dione, 3-aryl-1-pyrrolidinyl-2,5-dione, 1-pipéridinyl-2,6-dione, 3-alkyl-1-pipéridinyl-2,6-dione, 3-aryl-1-pipéridinyl-2,6-dione, 2H-iso-indol-2-yl-1,3-dione, hexahydro-2H-iso-indol-2-yl-1,3-dione, 2,5-dihydro-2,5-dioxo-1H-pyrrol-1-yle, 1,1,3-trioxo-1,2-benzisothiazol-2(3H)-yle ou 1,3-dihydro-1,3-dioxo-2H-benz[de]isoquinoléin-2-yle;

$R_3$ est un atome d'hydrogène, un radical alkyle, aryle ou arylalkyle, ou un groupement hydrolysable protecteur de la fonction acyle;

$R_4$ est un atome d'hydrogène ou un radical alkyle, alkylthio ou trifluorométhyle;

$R_5$ est un atome d'hydrogène ou un radical alkyle ou arylalkyle;

n est égal à 0, 1 ou 2; et

p est égal à 1 ou 2;

"aryle", soit en tant que tel soit en tant que partie d'un groupement plus important, étant un radical phényle, 1-naphtyle, 2-naphtyle ou phényle substitué par un ou plusieurs atomes d'halogène ou radicaux alkyle, alcoxy, alkylthio, hydroxy, alcanoyle, nitro, amine, dialkylamine, phényle ou trifluorométhyle;

"cycloalkyle", soit en tant que tel soit en tant que partie d'un groupement plus important, désignant des radicaux cycloalkyle qui ont de 3 à 7 atomes de carbone;

"alkyle", soit en tant que tel soit en tant que partie d'un groupement plus important, désignant des radicaux ayant de 1 à 8 atomes de carbone;

"alcoxy", soit en tant que tel soit en tant que partie d'un groupement plus important, désignant des radicaux qui ont de 1 à 8 atomes de carbone;

"halogène", soit en tant que tel soit en tant que partie d'un groupement plus important, étant le flour, le chlore, le brome ou l'iode;

"alcanoyle", soit en tant que tel soit en tant que partie d'un groupement plus important, désignant des radicaux qui ont de 1 à 9 atomes de carbone.

2. Composé selon la revendication 1, dans lequel $R_3$ est un atome d'hydrogène.

3. Composé selon la revendication 1, dans lequel $R_3$ est un groupement protecteur hydrolysable qui est choisi dans le groupe comprenant les radicaux alcanoyle et arylcarbonyle tels qu'ils sont définis dans la revendication 1.

4. Composé selon la revendication 1, dans lequel $R_4$ est un atome d'hydrogène.

5. Composé selon la revendication 1, dans lequel $R_4$ est un radical alkyle tel qu'il est défini dans la revendication 1.

6. Composé selon la revendication 1, dans lequel $R_4$ est un radical alkylthio tel qu'il est défini dans la revendication 1.

7. Composé selon la revendication 1, dans lequel $R_4$ est un radical trifluorométhyle.

8. Composé selon la revendication 1, dans lequel n est égal à 1.

9. Composé selon la revendication 1, dans lequel n est égal à 2.

10. Composé selon la revendication 1, dans lequel p est égal à 1.

11. Composé selon la revendication 1, dans lequel p est égal à 2.

12. Composé selon la revendication 1, dans lequel $R_5$ est un atome d'hydrogène.

13. Composé selon la revendication 1, dans lequel n est égal à 1, p est égal à 1, $R_4$ est un radical méthyle, et $R_5$ est un atome d'hydrogène.

14. Composé selon la revendication 1, qui est la [1(S),4S] - 4 - (benzoylamino) - 1 - (3 - mercapto - 2 - méthyl - 1 - oxopropyl) - L - proline.

15. Composé selon la revendication 1, qui est la [1(S),4S] - 1 - [3 - acétylthio) - 2 - méthyl - 1 - oxopropyl] - 4 - (1,3 - dihydro - 1,3 - dioxo - 2H - iso - indol - 2 - yl) - L - proline.

16. Composé selon la revendication 1, dans lequel —$NR_1R_2$ est un groupement azide.

17. Composé selon la revendication 16, dans lequel $R_3$ est un atome d'hydrogène.

18. Composé selon la revendication 16, dans lequel n est égal à 1, p est égal à 1 et $R_2$ est un radical méthyle.

19. Composé selon la revendication 1, qui est l'ester méthylique de la [1(S),4S] - 4 - azido - 1 - [3 - (benzoylthio) - 2 - méthyl - 1 - oxopropyl] - L - proline.

20. Composé selon la revendication 1, qui est la [1(S),4S] - 4 - azido - 1 - (3 - mercapto - 2 - méthyl - 1 - oxopropyl) - L - proline.